# EUROPEAN PATENT SPECIFICATION

(11) **EP 3 842 017 B1**
(45) Date of publication and mention of the grant of the patent: **17.01.2024**
(21) Application number: 19219708.5
(22) Date of filing: 24.12.2019
(51) Int. Cl.: A61F 13/505, A61F 13/70, A61F 13/78, A61F 13/80

(54) **ABSORBENT ARTICLE COMPRISING DISPOSABLE INSERT**
ABSORPTIONSARTIKEL MIT EINWEGEINLAGE
ARTICLE ABSORBANT COMPRENANT UN INSERT JETABLE

(43) Date of publication of application: 30.06.2021
(73) Proprietor: Ontex BV, 9255 Buggenhout (BE); Ontex Group NV, 9320 Erembodegem (BE)
(72) Inventor: AKYILDIZ, Özgür, 9320 Aalst (BE)
(74) Representative: Larangé, Françoise

(56) References cited:
- EP-A1- 2 699 213
- US-A1- 2007 021 728
- US-A1- 2012 209 237
- US-A1- 2014 005 621
- US-A1- 2016 058 631

## Description

### TECHNICAL FIELD

The disclosure pertains to the technical field of absorbent personal hygiene products and in particular disposable absorbent products such as baby pants comprising a disposable insert and a re-usable outer cover.

### BACKGROUND

Absorbent articles for personal hygiene such as disposable diapers, feminine protection pads and adult incontinence undergarments, are designed to absorb and contain body exudates, in particular but not limited to urine. These absorbent articles usually comprise several layers having different functions, for example a topsheet, a backsheet and in-between an absorbent core, among other layers. The absorbent core's function is to absorb and retain the exudates for a prolonged amount of time, for example overnight for a diaper, minimize re-wet to keep the wearer dry and avoid soiling of clothes or bed sheets.

Absorbent cores can expand several times their initial volumes when wet. It is desirable that the cores in this expanded state maintain their structural integrity and do not break or burst even when submitted to a shock such as a child sitting heavily on his diaper. It is generally also desirable that absorbent cores should be thin (at least when dry) and require as little material as possible for costs and environmental reasons.

The prior art contains a number of examples of hybrid-type articles where at least some components thereof are re-usable and washable and others that are disposable. Most however relate to a reusable insert and disposable outer cover, and some few focus on vice versa.

For example, US8,814,843 relates to reusable diaper liner devices, systems, and methods. In various embodiments, a reusable diaper liner can include an absorption layer that includes a first surface and a second surface, opposite the first surface. The reusable diaper liner also can include a waterproof layer that has a first surface and a second surface, opposite the first surface. The second surface of the absorption layer and the first surface of the waterproof layer can be positioned adjacent to each other and the reusable diaper liner can be configured to fit inside a diaper body. As another example, US20120209237 A1 relates to a fastening and carrying device for a disposable absorbent incontinence pad, having a hip belt that can be closed detachably onto itself by means of belt closure elements and thus forming a continuous hip opening in the circumferential hip direction, to which belt the incontinence pad is detachably fixable, so that it can be worn in the crotch region of the user and after use removed from the hip belt again and discarded. Disadvantages of such devices are several and include intricate insert removal operations, and risk of soiling clothes and other surfaces during the removal thereof.

There thus still remains a need to provide hybrid diaper/pants that are specifically designed for providing a cost-effective and environmentally friendly article that more particularly simplifies the removal of a disposable insert from a re-usable outer cover for single-handed manipulation thereof.

### SUMMARY OF THE INVENTION

In a first aspect, the disclosure relates to an absorbent assembly comprising: an elastic outer cover comprising a front and back waist region having uppermost and lowermost edges, and a crotch region interposed between the front and back waist region, wherein the uppermost edges of the waist region form a waist opening and the lowermost edges of the waist region together with lateral extremities of the crotch region form two oppositely disposed leg openings; and a disposable absorbent insert comprising a liquid permeable topsheet, a liquid impermeable backsheet, and an absorbent core interposed between said topsheet and said backsheet, wherein a skin-facing surface of said outer cover comprises one or more fastening surfaces and wherein a garment-facing surface of the absorbent insert comprises one or more fastening surfaces arranged to releasably engage with the one or more fastening surfaces of said outer cover, and wherein said crotch region comprises one or more re-fastenable members such that said crotch region is unitary when said re-fastenable members are fastened and that said crotch region is separated into two crotch halfs when said re-fastenable members are unfastened to form a crotch-release opening, and in that the disposable absorbent insert may be single-handedly removed from said elastic outer cover from said crotch-release opening.

In a second aspect, the disclosure relates to a disposable absorbent insert, for use in an absorbent assembly, comprising: a liquid permeable topsheet, a liquid impermeable backsheet, and an absorbent core interposed between said topsheet and said backsheet; a pair of longitudinal cuffs extending along a length of the disposable absorbent insert; and a transversal barrier extending between oppositely extending lateral edges of said disposable absorbent insert arranged on at least one end thereof, wherein said transversal barrier comprises indicia such that said disposable absorbent insert may be joined to the elastic outer cover of said absorbent assembly in the correct front-to-back orientation.

In a third aspect, the disclosure relates to a method of removing a disposable absorbent insert from an elastic outer cover comprising the steps of: providing an elastic outer cover comprising a front and back waist region having uppermost and lowermost edges, and a crotch region interposed between the front and back waist region, wherein the uppermost edges of the waist region form a waist opening and the lowermost edges of the waist region together with lateral extremities of the crotch region form two oppositely disposed leg openings, and wherein said crotch region comprises one or more re-fastenable members such that said crotch region is unitary when said re-fastenable members are fastened and that said crotch region is separated into two crotch halfs when said re-fastenable members are unfastened to form a crotch-release opening; providing a disposable absorbent insert comprising a liquid permeable topsheet, a liquid impermeable backsheet, and an absorbent core interposed between said topsheet and said backsheet, wherein a skin-facing surface of said outer cover is releasably joined to garment-facing surface of the absorbent insert; opening said re-fastenable members; pulling at least one of the two crotch halfs away from a garment-facing surface of the disposable absorbent insert to expose at least a portion thereof; single-handedly grab the garment-facing surface of the disposable absorbent insert with a thumb at a position proximal to one end and distal to another end of said insert, and with the remaining fingers at an opposite position distal to one end and proximal to the other end of said insert; squeeze the thumb and remaining fingers together; and substantially concurrently extracting the absorbent insert from the crotch-release opening.

### DESCRIPTION OF FIGURES

Fig. 1A-C illustrates a schematic of an article assembly according to an embodiment of the disclosure.
Fig. 2 illustrates a schematic of an insert according to an embodiment of the disclosure.
Fig.3 illustrates a schematic of an insert according to an embodiment of the disclosure.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation or element referred to.

The recitation of numerical ranges by endpoints includes all numbers and fractions subsumed within that range, as well as the recited endpoints, except where otherwise explicitly stated by disclaimer and the like.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, the term "associated or joined or adhered" encompasses, unless expressly stated, configurations in which e.g. a top sheet is directly joined to a back sheet by affixing the top sheet directly to the back sheet, and also configurations wherein the top sheet is joined to the back sheet by affixing the top sheet to intermediate members which in turn are affixed to the back sheet (i.e. indirectly joining). Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The terms "back portion", "back section" and "rear back section" are used herein as synonyms and refer to the area of the absorbent article which is contact with the back of the wearer when the absorbent article is worn.

The term "backsheet" refers to a material forming a liquid impermeable cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The terms "front portion", "belly section" and "front belly section" are used herein as synonyms and refer to the area of the absorbent article which is contact with the belly of the wearer when the absorbent article is worn.

As used herein, the "body-facing" or "bodyside" or "skin-facing" surface means that surface of the article or component which is intended to be disposed toward or placed adjacent to the body of the wearer during ordinary use, while the "outward", "outward-facing", "garment-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, an absorbent article can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

An absorbent article can comprise leg containment gaskets (also broadly referred to as "cuffs"). Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" or "fully stretched state" or "extended state" is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "nonwoven substrate/fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

Use of the term "substrate" includes, but is not limited to, woven or nonwoven webs, porous films, ink permeable films, paper, composite structures, or the like.

Superabsorbent materials (e.g. superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to 100% by weight. Typically, the superabsorbent material, when present, will be included in an amount of from about 30% to about 70% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

The term "topsheet" refers to a liquid permeable material sheet forming the inner cover of the absorbent article and which in use is placed in direct contact with the skin of the wearer. The topsheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction (i.e. is parallel to the x-axis).

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 5-60 µm, preferably from 10-30 µm. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

Embodiments according to the disclosure will now be described. It is understood that technical features described in one or more embodiments maybe combined with one or more other embodiments without departing from the intention of the disclosure and without generalization therefrom.

### THE ABSORBENT ASSEMBLY

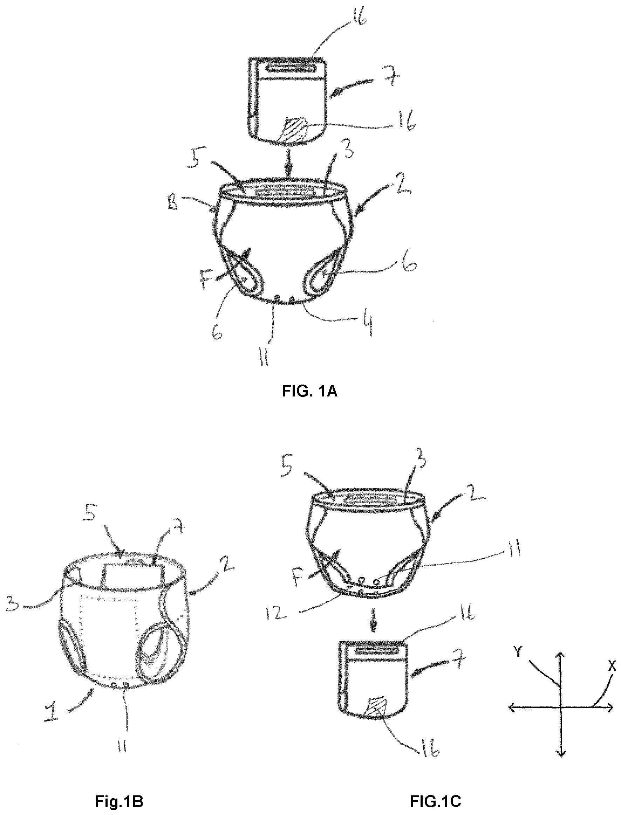

As exemplified in Figs.1A-C, the disclosure relates to an absorbent assembly (1) comprising: an elastic outer cover (2) comprising a front (F) and back (B) waist region (3) having uppermost and lowermost edges, and a crotch region (4) interposed between the front and back waist region, wherein the uppermost edges of the waist region (3) form a waist opening (5) and the lowermost edges of the waist region (3) together with lateral extremities of the crotch region (4) form two oppositely disposed leg openings (6); and a disposable absorbent insert (7) comprising a liquid permeable topsheet (8), a liquid impermeable backsheet (9), and an absorbent core (10) interposed between said topsheet (8) and said backsheet (9), wherein a skin-facing surface of said outer cover (2) comprises one or more fastening surfaces and wherein a garment-facing surface of the absorbent insert (7) comprises one or more fastening surfaces arranged to releasably engage with the one or more fastening surfaces of said outer cover (2), and wherein said crotch region (4) comprises one or more re-fastenable members (11) such that said crotch region (4) is unitary when said re-fastenable members (11) are fastened and that said crotch region (4) is separated into two crotch halfs when said re-fastenable members (11) are unfastened to form a crotch-release opening (12), and in that the disposable absorbent insert (7) may be single-handedly removed from said elastic outer cover (2) from said crotch-release opening (12). Advantageously, this allows a subject to remove the insert without soiling the elastic outer cover that may be re-used by inserting a new insert therein, even more advantageously this operation may be carried out with a single hand thus allowing the subject to handle the baby with the other hand and or doing other operations concurrently.

In an embodiment, the re-fastenable members (11) are mechanical fasteners, preferably in the form of more than one snap fasteners. Such allow for multiple secure opening/re-closing over time for effective re-usability of the outer cover.

The outer cover may be constructed with woven textile material comprising one or more elastics and/or comprising an elastic fabric that provides it with stretch properties similar to underwear for added comfort. The outer cover may further be washable.

In an embodiment, the elastic outer cover (2) comprises corresponding fastening surfaces (to the disposable absorbent insert as will be further described in embodiments hereinbelow) with the proviso that within the crotch region (4) said respective fastening surface is present only on one of the two crotch halfs, preferably said corresponding fastening surfaces comprising one or more woven, nonwoven or loop material.

### THE DISPOSABLE ABSORBENT INSERT

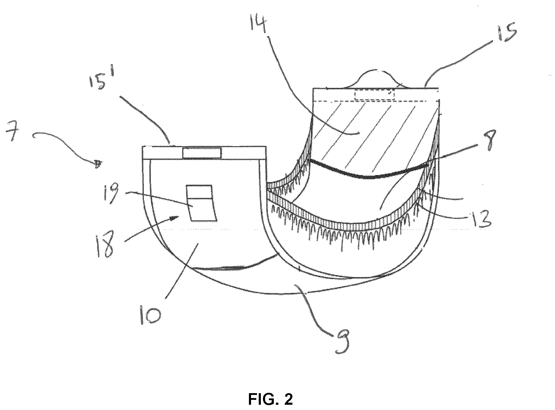

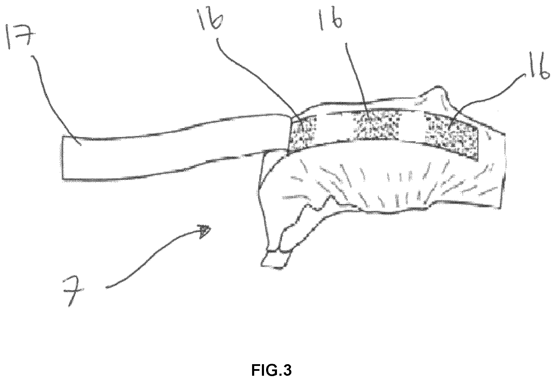

Referring to Figs.2-3, disposable absorbent inserts (7) according to the disclosure and for use in an absorbent assembly (1) described herein comprise: a liquid permeable topsheet (8), a liquid impermeable backsheet (9), and an absorbent core (10) interposed between said topsheet (8) and said backsheet (9); a pair of longitudinal cuffs (13) extending along a length of the disposable absorbent insert (7); and a transversal barrier (14), typically extending in a direction substantially parallel to the transverse axis x, extending between oppositely extending lateral edges of said disposable absorbent insert (7), arranged on at least one end (15) thereof, wherein said transversal barrier (14) comprises indicia such that said disposable absorbent insert (7) may be joined to the elastic outer cover (2) of said absorbent assembly (1) in the correct front-to-back orientation. Advantageously this allows the user to correctly apply the insert into the outer cover (2) in the correct orientation. Indeed absorbent inserts are typically designed with graded absorbent properties along the length of the core and thus correctly orienting the insert is important to ensure efficacious exudate collection.

The absorbent core (10) may comprise absorbent material selected from the group consisting of cellulose fluff, cellulose fibers, and superabsorbent polymer particles.

In an embodiment, said insert (7) comprises a transversal barrier (14) having indicia in the form of a color such that the insert (7) may be joined to the elastic outer cover (2) of said absorbent assembly (1) on the back portion thereof.

Preferably, said insert (7) comprises two transversal barriers (14) oppositely disposed on each ends (15, 15') with the proviso that the barrier (14) proximal to the end (15), that is to be positioned on a back portion of the elastic cover (2) of said absorbent assembly (1), comprises indicia that is different to that of the oppositely disposed barrier, wherein said indicia is selected from the group consisting of a color and length, preferably wherein said indicia is the length of said barriers and wherein said barrier (14) proximal to said end (15) has a longer length, preferably being at least 1.5 times the length, of the other oppositely disposed barrier. Advantageously this allows to provide an orientation indication and also a functional barrier to liquid with the same components, indeed the barriers arranged as described herein act synergistically to provide liquid leakage prevention in the waist region (particularly important when using a foreign insert in a reusable outer cover that is not integral with said insert) and at the same time an indication as to correct orientation of the insert for correct assembly and efficacious exudate collection.

In an embodiment, a garment-facing surface of the absorbent insert (7) comprises a plurality fastening surfaces arranged to releasably engage with the one or more fastening surfaces of said outer cover (2), wherein said plurality of fastening surfaces are in the form of strips (16) each being separated along the length of said absorbent insert (7), more preferably being in the form of at least three strips (16) arranged proximal to the ends (15. 15') of said insert (7) and at a position therebetween. Advantageously this allows for secure mounting of the insert on the outer cover whilst limiting cost compared to for example having a continuous fastening surface.

Preferably, the plurality fastening surfaces are covered with a single release strip (17) that must be peeled off in order to join the disposable absorbent insert (7) to the elastic outer cover (2). Advantageously this prevents the fastening surface from becoming soiled and/or reduce its adhering efficacy when storing or prior to joining to the outer cover and allows for simple single-handed removal thereof prior to attachment.

Preferably, the plurality fastening surfaces comprise an adhesive or mechanical fasteners such as hooks, preferably adhesive.

In a preferred embodiment, a garment-facing surface of the absorbent insert (7) comprises a single fastening surface arranged to releasably engage with the one or more fastening surfaces of said outer cover (2), wherein said single fastening surface is in the form of a nonwoven having basis weight of from 10 to 35 g/m² that is laminated onto a garment-facing surface of the backsheet (9) of said absorbent insert (7) and is arranged to engage with a plurality of mechanical fasteners comprising hooks arranged on a skin-facing surface of the outer cover (2). Advantageously, when this is combined with one or more hooks present on a skin-facing surface of the outer cover allows for good attachment of the insert whiles at the same time providing a softer touch and feel to the garment-facing side of the backsheet.

Preferably, the garment-facing surface of the disposable absorbent insert (7), preferably the garment-facing surface of the backsheet (9) thereof, comprises two oppositely disposed pockets (18) proximal each end (15, 15') and separated from each other in a length direction of said insert (7), and arranged to receive a thumb of a subject in one of said pockets (18) and the rest of the fingers of the same hand of the subject in the other of said pockets (18) such that the insert may be single-handedly removed from the outer cover (2). Advantageously this aids the user to correctly single-handedly grip the insert and works synergistically with the designed release opening of the outer cover to allow a simple and effective single-handed removal of the insert once soiled.

In an embodiment, each pocket (18) comprises a substrate joined to a garment facing surface of said insert (7), preferably the garment-facing surface of the backsheet (9) thereof, having an open end distal from respective end (15, 15') and proximal to a transverse centreline of said insert (7) that divides said insert (7) into two halves, preferably wherein said open end is the only open end of each said pockets (18). Advantageously this allows for constructing pockets having a relatively large surface area to comfortably accommodate respective portions of an user's hand.

In an embodiment, the substrate comprises a nonwoven substrate having a basis weight of greater than 10 g/m² and wherein said substrate is joined to a garment facing surface of said insert (7), preferably the garment-facing surface of the backsheet (9) thereof, by mechanical or adhesive bonding substantially along a perimeter thereof except for the open end distal from respective end (15, 15') and proximal to a transverse centreline of said insert (7). This arrangement has the advantage of providing sufficient mechanical resistance to prevent tearing when the subject inserts his/her hand within respective pockets.

In an embodiment, the insert (7) further comprises a disposal tape (19) that may be arranged on a garment-facing surface of said insert (7), preferably of the pocket (18), such that once the insert is single-handedly removed it may be rolled up and joined to retain any exudates in an enclosed manner therein. Preferably the disposal tape (19) is arranged proximal to an end (15') of the insert (7), even more preferably is arranged only proximal to said end (15'). Advantageously, this allows the insert, once removed, to be rolled up starting from one end (15) towards the other end (15') and then simply joining the garment-facing surface of the insert (7) to said disposal tape (19).

### THE METHOD OF DINGLE-HANDED MANIPULATION

Referring to Fig.1C, a method of removing a disposable absorbent insert (7) from an elastic outer cover (2) comprises the steps of: providing an elastic outer cover (2) comprising a front and back waist region (3) having uppermost and lowermost edges, and a crotch region (4) interposed between the front and back waist region, wherein the uppermost edges of the waist region (3) form a waist opening (5) and the lowermost edges of the waist region (3) together with lateral extremities of the crotch region (4) form two oppositely disposed leg openings (6), and wherein said crotch region (4) comprises one or more re-fastenable members (11) such that said crotch region (4) is unitary when said re-fastenable members (11) are fastened and that said crotch region (4) is separated into two crotch halfs when said re-fastenable members (11) are unfastened to form a crotch-release opening (12); providing a disposable absorbent insert (7) comprising a liquid permeable topsheet (8), a liquid impermeable backsheet (9), and an absorbent core (10) interposed between said topsheet (8) and said backsheet (9), wherein a skin-facing surface of said outer cover (2) is releasably joined to garment-facing surface of the absorbent insert (7); opening said re-fastenable members (11); pulling at least one of the two crotch halfs away from a garment-facing surface of the disposable absorbent insert (7) to expose at least a portion thereof; single-handedly grab the garment-facing surface of the disposable absorbent insert (7) with a thumb at a position proximal to one end (15') and distal to another end (15) of said insert (7), and with the remaining fingers at an opposite position distal to one end (15') and proximal to the other end (15) of said insert (7); squeeze the thumb and remaining fingers together; and substantially concurrently extracting the absorbent insert (7) from the crotch-release opening (12). Advantageously this method allows for single-handed removal of the insert whilst containing the exudate material in a cup-shape throughout the entire removal procedure and thus limit soiling of clothes and/or other surfaces.

Preferably, the thumb and the remaining fingers of a subject are inserted into oppositely disposed pockets (18), on the garment-facing surface of the absorbent insert (7), prior to the step of squeezing the thumb and remaining fingers together. Advantageously this arrangement allows for better location of the fingers of the user and provides added support and grip to allow the removal operation to be carried out successfully.

In an embodiment, as shown in Fig.1A, the method of inserting the insert (7) onto said outer cover (2) is different from the method of removal thereof (e.g. as described above). Preferably wherein the method of inserting the insert comprises the steps of inserting the insert (7) through the waist opening (5), typically whilst the re-fastenable members (11) are in a fastened state, and joining the garment-facing side of said insert (7) to the skin-facing side of said outer cover (2).

It is supposed that the present invention is not restricted to any form of realization described previously and that some modifications can be added to the presented example of fabrication without reappraisal of the appended claims.

## Claims

1. An absorbent assembly (1) comprising:
an elastic outer cover (2) comprising a front (F) and back (B) waist region (3) having uppermost and lowermost edges, and a crotch region (4) interposed between the front and back waist region, wherein the uppermost edges of the waist region (3) form a waist opening (5) and the lowermost edges of the waist region (3) together with lateral extremities of the crotch region (4) form two oppositely disposed leg openings (6); and
a disposable absorbent insert (7) comprising a liquid permeable topsheet (8), a liquid impermeable backsheet (9), and an absorbent core (10) interposed between said topsheet (8) and said backsheet (9),
wherein a skin-facing surface of said outer cover (2) comprises one or more fastening surfaces and wherein a garment-facing surface of the absorbent insert (7) comprises one or more fastening surfaces arranged to releasably engage with the one or more fastening surfaces of said outer cover (2), and **characterized in that** said crotch region (4) comprises one or more re-fastenable members (11) such that said crotch region (4) is unitary when said re-fastenable members (11) are fastened and that said crotch region (4) is separated into two crotch halfs when said re-fastenable members (11) are unfastened to form a crotch-release opening (12), and **in that** the disposable absorbent insert (7) may be single-handedly removed from said elastic outer cover (2) from said crotch-release opening (12).

2. A disposable absorbent insert (7) for use in an absorbent assembly (1) according to Claim 1 comprising:
a liquid permeable topsheet (8), a liquid impermeable backsheet (9), and an absorbent core (10) interposed between said topsheet (8) and said backsheet (9);
a pair of longitudinal cuffs (13) extending along a length of the disposable absorbent insert (7); and
a transversal barrier (14) extending between oppositely extending lateral edges of said disposable absorbent insert (7) arranged on at least one end (15) thereof,
wherein said transversal barrier (14) comprises indicia such that said disposable absorbent insert (7) may be joined to the elastic outer cover (2) of said absorbent assembly (1) in the correct front-to-back orientation.

3. An absorbent assembly (1) or disposable absorbent insert (7) according to any of the preceding Claims wherein said insert (7) comprises a transversal barrier (14) having indicia in the form of a color such that the insert (7) may be joined to the elastic outer cover (2) of said absorbent assembly (1) on the back portion thereof.

4. An absorbent assembly (1) or disposable absorbent insert (7) according to any of the preceding Claims wherein said insert (7) comprises two transversal barriers (14) oppositely disposed on each ends (15, 15') with the proviso that the barrier (14) proximal to the end (15), that is to be positioned on a back portion of the elastic cover (2) of said absorbent assembly (1), comprises indicia that is different to that of the oppositely disposed barrier, wherein said indicia is selected from the group consisting of a color and length, preferably wherein said indicia is the length of said barriers and wherein said barrier (14) proximal to said end (15) has a longer length, preferably being at least 1.5 times the length, of the other oppositely disposed barrier.

5. An absorbent assembly (1) or disposable absorbent insert (7) according to any of the preceding Claims wherein the re-fastenable members (11) are mechanical fasteners, preferably in the form of more than one snap fasteners.

6. An absorbent assembly (1) or disposable absorbent insert (7) according to any of the preceding Claims wherein a garment-facing surface of the absorbent insert (7) comprises a plurality fastening surfaces arranged to releasably engage with the one or more fastening surfaces of said outer cover (2), wherein said plurality of fastening surfaces are in the form of strips (16) each being separated along the length of said absorbent insert (7), more preferably being in the form of at least three strips (16) arranged proximal to the ends (15. 15') of said insert (7) and at a position therebetween.

7. An absorbent assembly (1) or disposable absorbent insert (7) according to Claim 6 wherein the plurality fastening surfaces are covered with a single release strip (17) that must be peeled off in order to join the disposable absorbent insert (7) to the elastic outer cover (2).

8. An absorbent assembly (1) or disposable absorbent insert (7) according to Claims 6 or 7 wherein the plurality fastening surfaces comprise an adhesive or mechanical fasteners such as hooks, preferably adhesive.

9. An absorbent assembly (1) or disposable absorbent insert (7) according to Claims 6 to 8 wherein the elastic outer cover (2) comprises corresponding fastening surfaces with the proviso that within the crotch region (4) said respective fastening surface is present only on one of the two crotch halfs, preferably said corresponding fastening surfaces comprising one or more woven, nonwoven or loop material.

10. An absorbent assembly (1) or disposable absorbent insert (7) according to Claims 1 to 5 wherein a garment-facing surface of the absorbent insert (7) comprises a single fastening surface arranged to releasably engage with the one or more fastening surfaces of said outer cover (2), wherein said single fastening surface is in the form of a nonwoven having basis weight of from 10 to 35 g/m² that is laminated onto a garment-facing surface of the backsheet (9) of said absorbent insert (7) and is arranged to engage with a plurality of mechanical fasteners comprising hooks arranged on a skin-facing surface of the outer cover (2).

11. An absorbent assembly (1) or disposable absorbent insert (7) according to any of the preceding Claims wherein the garment-facing surface of the disposable absorbent insert (7), preferably the garment-facing surface of the backsheet (9) thereof, comprises two oppositely disposed pockets (18) proximal each end (15, 15') and separated from each other in a length direction of said insert (7), and arranged to receive a thumb of a subject in one of said pockets (18) and the rest of the fingers of the same hand of the subject in the other of said pockets (18) such that the insert may be single-handedly removed from the outer cover (2).

12. An absorbent assembly (1) or disposable absorbent insert (7) according to Claim 11 wherein each pocket (18) comprises a substrate joined to a garment facing surface of said insert (7), preferably the garment-facing surface of the backsheet (9) thereof, having an open end distal from respective end (15, 15') and proximal to a transverse centreline of said insert (7) that divides said insert (7) into two halves, preferably wherein said open end is the only open end of each said pockets (18).

13. An absorbent assembly (1) or disposable absorbent insert (7) according to Claim 12 wherein the substrate comprises a nonwoven substrate having a basis weight of greater than 10 g/m² and wherein said substrate is joined to a garment facing surface of said insert (7), preferably the garment-facing surface of the backsheet (9) thereof, by mechanical or adhesive bonding substantially along a perimeter thereof except for the open end distal from respective end (15, 15') and proximal to a transverse centreline of said insert (7).

## Patentansprüche

1. Absorptionseinheit (1), die Folgendes umfasst:
eine elastische äußere Abdeckung (2), umfassend eine vordere (F) und hintere (B) Hüftregion (3), die oberste und unterste Kanten aufweist, und eine Schrittregion (4), die zwischen der vorderen und hinteren Hüftregion angebracht ist, wobei die obersten Kanten der Hüftregion (3) eine Hüftöffnung (5) bilden und die untersten Kanten der Hüftregion (3) zusammen mit seitlichen Enden der Schrittregion (4) zwei einander gegenüber angeordnete Beinöffnungen (6) bilden; und
eine absorbierende Einwegeinlage (7) umfassend eine flüssigkeitsdurchlässige obere Folie (8), eine flüssigkeitsundurchlässige hintere Folie (9) und einen absorbierenden Kern (10), der zwischen der oberen Folie (8) und der hinteren Folie (9) angebracht ist,
wobei eine auf die Haut gerichtete Fläche der äußeren Abdeckung (2) eine oder mehrere Befestigungsflächen umfasst, und wobei eine auf die Kleidung gerichtete Fläche der absorbierenden Einlage (7) eine oder mehrere Befestigungsflächen umfasst, die angeordnet sind, um mit der einen oder mehreren Befestigungsflächen der äußeren Abdeckung (2) entfernbar einzugreifen, und **dadurch gekennzeichnet, dass** die Schrittregion (4) ein oder mehrere wiederbefestigbaren Elemente (11) umfasst, so dass die Schrittregion (4) einheitlich ist, wenn die wiederbefestigbaren Elemente (11) befestigt werden, und dass die Schrittregion (4) in zwei Schritthälften unterteilt wird, wenn die wiederbefestigbaren Elemente (11) gelöst werden, um eine Schritt-Freigabeöffnung (12) zu bilden, und dadurch, dass die absorbierende Einwegeinlage (7) mit einer Hand von der elastischen äußeren Abdeckung (2) von der Schritt-Freigabeöffnung (12) entfernt werden kann.

2. Absorbierende Einwegeinlage (7) zur Verwendung in einer Absorptionseinheit (1) nach Anspruch 1, umfassend:
eine flüssigkeitsdurchlässige obere Folie (8), eine flüssigkeitsundurchlässige hintere Folie (9) und einen absorbierenden Kern (10), der zwischen der oberen Folie (8) und der hinteren Folie (9) angebracht ist,
ein Paar längs gerichteter Manschetten (13), die sich entlang einer Länge der absorbierenden Einwegeinlage (7) erstrecken; und
eine quer gerichtete Barriere (14), die sich zwischen sich einander gegenüber erstreckenden seitlichen Kanten der absorbierenden Einwegeinlage (7) erstreckt, die an mindestens einem Ende (15) davon angeordnet ist,
wobei die quer gerichtete Barriere (14) eine Anzeige umfasst, so dass die absorbierende Einwegeinlage (7) mit der elastischen äußeren Abdeckung (2) der Absorptionseinheit (1) in der korrekten Ausrichtung von vorne nach hinten verbunden werden kann.

3. Absorptionseinheit (1) oder absorbierende Einwegeinlage (7) nach einem der vorhergehenden Ansprüche, wobei die Einlage (7) eine quer gerichtete Barriere (14) umfasst, die eine Anzeige in Form einer Farbe aufweist, so dass die Einlage (7) mit der elastischen äußeren Abdeckung (2) der Absorptionseinheit (1) auf dem hinteren Abschnitt davon verbunden werden kann.

4. Absorptionseinheit (1) oder absorbierende Einwegeinlage (7) nach einem der vorhergehenden Ansprüche, wobei die Einlage (7) zwei quer gerichtete Barrieren (14) umfasst, die einander gegenüberliegend auf jedem Ende (15, 15') angeordnet sind, mit der Maßgabe, dass die Barriere (14) proximal zum Ende (15), die auf einem hinteren Abschnitt der elastischen Abdeckung (2) der Absorptionseinheit (1) positioniert werden soll, eine Anzeige umfasst, die verschieden von denjenigen der einander gegenüberliegend angeordneten Barriere sind, wobei die Anzeige ausgewählt ist aus der Gruppe, bestehend aus einer Farbe und Länge, vorzugsweise wobei die Anzeige die Länge der Barrieren ist, und wobei die Barriere (14) proximal zu dem Ende (15) eine größere Länge aufweist und vorzugsweise mindestens 1,5 Mal die Länge der anderen gegenüberliegend angeordneten Barriere ist.

5. Absorptionseinheit (1) oder absorbierende Einwegeinlage (7) nach einem der vorhergehenden Ansprüche, wobei die wiederbefestigbaren Elemente (11) mechanische Befestigungen sind, vorzugsweise in Form mehr als eines Druckknopfs.

6. Absorptionseinheit (1) oder absorbierende Einwegeinlage (7) nach einem der vorhergehenden Ansprüche, wobei eine auf die Kleidung gerichtete Fläche der absorbierenden Einlage (7) eine Vielzahl von Befestigungsflächen umfasst, die angeordnet sind, um mit der einen oder mehreren Befestigungsflächen der äußeren Abdeckung (2) entfernbar einzugreifen, wobei die Vielzahl von Befestigungsflächen die Form von Streifen (16) aufweisen, von denen jeder entlang der Länge der absorbierenden Einlage (7) unterteilt ist, vorzugsweise in Form von mindestens drei Streifen (16), die proximal zu den Enden (15, 15') der Einlage (7) und an einer Position dazwischen angeordnet sind.

7. Absorptionseinheit (1) oder absorbierende Einwegeinlage (7) nach Anspruch 6, wobei die Vielzahl von Befestigungsflächen mit einem einzigen Trennstreifen (17) bedeckt ist, der abgelöst werden muss, um die absorbierende Einwegeinlage (7) mit der elastischen äußeren Abdeckung (2) zu verbinden.

8. Absorptionseinheit (1) oder absorbierender Einwegeinlage (7) nach Anspruch 6 oder 7, wobei die Vielzahl von Befestigungsflächen einen Klebstoff oder mechanische Befestigungsmittel wie z. B. Haken, vorzugsweise Klebstoff umfasst.

9. Absorptionseinheit (1) oder absorbierende Einwegeinlage (7) nach Anspruch 6 bis 8, wobei die elastische äußere Abdeckung (2) entsprechende Befestigungsflächen umfasst, mit der Maßgabe, dass innerhalb der Schrittregion (4) die entsprechende Befestigungsfläche nur auf einer der zwei Schritthälften vorhanden ist, wobei vorzugsweise die entsprechenden Befestigungsflächen ein oder mehrere Webstoffe, Vlies- oder Schlaufenmaterialien umfassen.

10. Absorptionseinheit (1) oder absorbierende Einwegeinlage (7) nach Anspruch 1 bis 5, wobei eine auf die Kleidung gerichtete Fläche der absorbierenden Einlage (7) eine einzelne Befestigungsfläche umfasst, die angeordnet ist, um mit der einen oder mehreren Befestigungsflächen der äußeren Abdeckung (2) entfernbar einzugreifen, wobei die einzelne Befestigungsfläche die Form eines Vliesstoffs aufweist, der ein Basisgewicht von 10 bis 35 g/m² aufweist, der auf eine auf die Kleidung gerichtete Fläche der hinteren Folie (9) der absorbierenden Einlage (7) laminiert und angeordnet ist, um mit einer Vielzahl von mechanischen Befestigungsmitteln, umfassend Haken, die auf einer auf die Haut gerichteten Fläche der äußeren Abdeckung (2) angeordnet sind, einzugreifen.

11. Absorptionseinheit (1) oder absorbierende Einwegeinlage (7) nach einem der vorhergehenden Ansprüche, wobei die auf die Kleidung gerichtete Fläche der absorbierenden Einwegeinlage (7), vorzugsweise die auf die Kleidung gerichtete Fläche der hinteren Folie (9) davon, zwei einander gegenüber angeordnete Beutel (18) proximal zu jedem Ende (15, 15') und voneinander getrennt in einer Längsrichtung der Einlage (7) umfasst und angeordnet, um einen Daumen eines Subjekts in einem der Beutel (18) und den Rest der Finger der gleichen Hand des Subjekts im anderen der Beutel (18) aufzunehmen, so dass die Einlage mit einer Hand von der äußeren Abdeckung (2) entfernt werden kann.

12. Absorptionseinheit (1) oder absorbierende Einwegeinlage (7) nach Anspruch 11, wobei jeder Beutel (18) ein Substrat umfasst, das mit einer auf die Kleidung gerichteten Fläche der Einlage (7), vorzugsweise der auf die Kleidung gerichteten Fläche der hinteren Folie (9) davon, die ein offenes Ende distal zum entsprechenden Ende (15, 15') und proximal zu einer quer liegenden Mittellinie der Einlage (7) aufweist, die die Einlage (7) in zwei Hälften unterteilt, vorzugsweise wobei das offene Ende das einzige offene Ende jedes der Beutel (18) ist, verbunden ist.

13. Absorptionseinheit (1) oder absorbierende Einwegeinlage (7) nach Anspruch 12, wobei das Substrat ein Vliessubstrat umfasst, das ein Basisgewicht von mehr als 10 g/m² aufweist, und wobei das Substrat mit einer auf die Kleidung gerichteten Fläche der Einlage (7), vorzugsweise der auf die Kleidung gerichteten Fläche der hinteren Folie (9) davon, durch mechanische oder Klebstoffbindung im Wesentlichen entlang eines Umfangs davon, mit Ausnahme des offenen Endes distal zum entsprechenden Ende (15, 15') und proximal zu einer quer liegenden Mittellinie der Einlage (7), verbunden ist.

## Revendications

1. Ensemble absorbant (1) comprenant :
un revêtement externe élastique (2) comprenant une région de taille (3) avant (F) et arrière (B) ayant les bords les plus hauts et les plus bas, et une région d'entrejambe (4) intercalée entre les régions de taille avant et arrière, dans lequel les bords les plus hauts de la région de taille (3) forment une ouverture de taille (5) et les bords les plus bas de la région de taille (3) conjointement avec les extrémités latérales de la région d'entrejambe (4) forment deux ouvertures de jambe (6) disposées de manière opposée ; et
un insert absorbant jetable (7) comprenant une feuille supérieure perméable au liquide (8), une feuille inférieure imperméable au liquide (9) et un noyau absorbant (10) intercalé entre ladite feuille supérieure (8) et ladite feuille inférieure (9),
dans lequel une surface orientée vers la peau dudit revêtement externe (2) comprend une ou plusieurs surfaces de fixation et dans lequel une surface orientée vers le vêtement de l'insert absorbant (7) comprend une ou plusieurs surfaces de fixation agencées pour se mettre en prise, de manière amovible, avec les une ou plusieurs surfaces de fixation dudit revêtement externe (2), et **caractérisé en ce que** ladite région d'entrejambe (4) comprend un ou plusieurs éléments pouvant être refixés (11) de sorte que ladite région d'entrejambe (4) est unitaire lorsque lesdits éléments pouvant être refixés (11) sont fixés et que ladite région d'entrejambe (4) est séparée en deux moitiés d'entrejambe lorsque lesdits éléments pouvant être refixés (11) sont détachés pour former une ouverture de libération d'entrejambe (12) et **en ce que** l'insert absorbant jetable (7) peut être retiré d'une main dudit revêtement externe élastique (2) par ladite ouverture de libération d'entrejambe (12).

2. Insert absorbant jetable (7) destiné à être utilisé dans un ensemble absorbant (1) selon la revendication 1, comprenant :
une feuille supérieure perméable au liquide (8), une feuille inférieure imperméable au liquide (9) et un noyau absorbant (10) intercalé entre ladite feuille supérieure (8) et ladite feuille inférieure (9) ;
une paire de revers longitudinaux (13) s'étendant le long d'une longueur de l'insert absorbant jetable (7) ; et
une barrière transversale (14) s'étendant entre des bords latéraux s'étendant de manière opposée dudit insert absorbant jetable (7) agencé sur au moins l'une de ses extrémités (15),
dans lequel ladite barrière transversale (14) comprend des indices de sorte que ledit insert absorbant jetable (7) peut être assemblé au revêtement externe élastique (2) dudit ensemble absorbant (1) dans la bonne orientation avant-arrière.

3. Ensemble absorbant (1) ou insert absorbant jetable (7) selon l'une quelconque des revendications précédentes, dans lequel ledit insert (7) comprend une barrière transversale (14) ayant des indices se présentant sous la forme d'une couleur de sorte que l'insert (7) peut être assemblé au revêtement externe élastique (2) dudit ensemble absorbant (1) sur sa partie arrière.

4. Ensemble absorbant (1) ou insert absorbant jetable (7) selon l'une quelconque des revendications précédentes, dans lequel ledit insert (7) comprend deux barrières transversales (14) disposées de manière opposée sur chaque extrémité (15, 15') sous réverse que la barrière (14) à proximité de l'extrémité (15), qui doit être positionnée sur une partie arrière du revêtement élastique (2) dudit ensemble absorbant (1), comprenne des indices qui sont différents de ceux de la barrière disposée de manière opposée, dans lequel lesdits indices sont sélectionnés dans le groupe comprenant une couleur et une longueur, de préférence dans lequel lesdits indices sont la longueur desdites barrières et dans lequel ladite barrière (14) à proximité de ladite extrémité (15) a une plus longue longueur, qui représente de préférence au moins 1,5 fois la longueur de l'autre barrière disposée, de manière opposée.

5. Ensemble absorbant (1) ou insert absorbant jetable (7) selon l'une quelconque des revendications précédentes, dans lequel les éléments pouvant être refixés (11) sont des fixations mécaniques, se présentant de préférence sous la forme de plus d'un bouton-pression.

6. Ensemble absorbant (1) ou insert absorbant jetable (7) selon l'une quelconque des revendications précédentes, dans lequel une surface orientée vers le vêtement de l'insert absorbant (7) comprend une pluralité de surfaces de fixation agencées pour se mettre en prise, de manière amovible, avec les une ou plusieurs surfaces de fixation dudit revêtement externe (2), dans lequel ladite pluralité de surfaces de fixation se présentent sous la forme de bandes (16) qui sont chacune séparées le long de la longueur dudit insert absorbant (7), encore de préférence qui se présentent sous la forme d'au moins trois bandes (16) agencées à proximité des extrémités (15, 15') dudit insert (7) et dans une position entre elles.

7. Ensemble absorbant (1) ou insert absorbant jetable (7) selon la revendication 6, dans lequel la pluralité de surfaces de fixation sont recouvertes avec une seule bande antiadhésive (17) qui doit être décollée afin d'assembler l'insert absorbant jetable (7) au revêtement externe élastique (2).

8. Ensemble absorbant (1) ou insert absorbant jetable (7) selon les revendications 6 ou 7, dans lequel la pluralité de surfaces de fixation comprend un adhésif ou des fixations mécaniques comme des crochets, de préférence de l'adhésif.

9. Ensemble absorbant (1) ou insert absorbant jetable (7) selon les revendications 6 à 8, dans lequel le revêtement externe élastique (2) comprend des surfaces de fixation correspondantes sous réserve que dans la région d'entrejambe (4), ladite surface de fixation respective n'est présente que sur l'une des deux moitiés d'entrejambe, de préférence lesdites surfaces de fixation correspondantes comprenant un ou plusieurs éléments parmi un matériau tissé, un matériau non tissé ou un matériau à boucles.

10. Ensemble absorbant (1) ou insert absorbant jetable (7) selon les revendications 1 à 5, dans lequel une surface orientée vers le vêtement de l'insert absorbant (7) comprend une seule surface de fixation agencée pour se mettre en prise de manière amovible avec les une ou plusieurs surfaces de fixation dudit revêtement externe (2), dans lequel ladite surface de fixation unique se présente sous la forme d'un élément non tissé ayant un poids de base de 10 à 35 g/m² qui est stratifié sur une surface orientée vers le vêtement de la feuille inférieure (9) dudit insert absorbant (7) et est agencée pour se mettre en prise avec une pluralité de fixations mécaniques comprenant des crochets agencés sur une surface orientée vers la peau du revêtement externe (2).

11. Ensemble absorbant (1) ou insert absorbant jetable (7) selon l'une quelconque des revendications précédentes, dans lequel la surface orientée vers le vêtement de l'insert absorbant jetable (7), de préférence la surface orientée vers le vêtement de sa feuille inférieure (9) comprend deux poches (18) disposées à l'opposé, à proximité de chaque extrémité (15, 15') et séparées l'une de l'autre dans une direction de longueur dudit insert (7), et agencées pour recevoir un pouce d'un sujet dans l'une desdites poches (18) et le reste des doigts de la même main du sujet dans l'autres desdites poches (18) de sorte que l'insert peut être retiré d'une main du revêtement externe (2).

12. Ensemble absorbant (1) ou insert absorbant jetable (7) selon la revendication 11, dans lequel chaque poche (18) comprend un substrat assemblé à une surface orientée vers le vêtement dudit insert (7), de préférence la surface orientée vers le vêtement de sa feuille inférieure (9), ayant une extrémité ouverte à distance de l'extrémité (15, 15') respective et à proximité d'une ligne médiane transversale dudit insert (7) qui divise ledit insert (7) en deux moitiés, de préférence dans lequel ladite extrémité ouverte est uniquement l'extrémité ouverte de chacune desdites poches (18).

13. Ensemble absorbant (1) ou insert absorbant jetable (7) selon la revendication 12, dans lequel le substrat comprend un substrat non tissé ayant un poids de base supérieur à 10 g/m² et dans lequel ledit substrat est assemblé à une surface orientée vers le vêtement dudit insert (7), de préférence la surface orientée vers le vêtement de sa feuille inférieure (9), par liaison mécanique ou adhésive sensiblement le long de son périmètre excepté pour l'extrémité ouverte à distance de l'extrémité (15, 15') respective et à proximité d'une ligne médiane transversale dudit insert (7).
